# FASCICULE DE BREVET EUROPEEN

(11) **EP 1 371 654 B1**
(45) Date de publication et mention de la délivrance du brevet: **14.06.2006**
(21) Numéro de dépôt: 03291203.2
(22) Date de dépôt: 21.05.2003
(51) Int. Cl.: C07F 7/08, C07F 7/21, C08G 77/388, A61K 8/00

(54) **Compositions photoprotectrices contenant des dérivés silaniques ou siloxaniques de phénylsulfone acrylonitrile, dérivées de phénylsulfone acrylonitrile et utilisation comme filtre UV**
Silan- oder Siloxan-Phenylsulfonacrylonitrilderivat-enthaltende Lichtschutzzusammensetzungen, Phenylsulfonacrylonitrilderivate und deren Verwendung als UV Filter
Sunscreen compositions containing silane or siloxane derivatives of phenylsulfon acrylonitrile, phenylsulfon acrylonitrile derivatives and their use as UV filter

(30) Priorité: 13.06.2002 FR 0207287
(43) Date de publication de la demande: 17.12.2003
(73) Titulaire: L'OREAL, 75008 Paris (FR)
(72) Inventeur: Richard, Hervé, 93420 Villepinte (FR); Breton, Philippe, 78150 Le Chesnay (FR)
(74) Mandataire: Miszputen, Laurent

(56) Documents cités:
- EP-A- 0 716 089

## Description

La présente invention concerne des compositions cosmétiques à usage topique, en particulier destinées à la photoprotection de la peau et/ou des cheveux, comprenant une quantité efficace d'au moins un dérivé silanique ou siloxanique de phénylsulfone acrylonitrile, de nouveaux dérivés silaniques ou siloxaniques de phenylsulfone acrylonitrile, ainsi que leurs utilisations en cosmétique comme filtres UV-A.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. Il est donc souhaitable de filtrer aussi le rayonnement UV-A.

De nombreux composés organiques destinés à la protection contre le rayonnement UV-A et/ou UV-B de la peau ont été proposés à ce jour.

La plupart d'entre eux sont des composés aromatiques absorbant le rayonnement UV dans la zone comprise entre 280 nm et 315 nm, ou dans la zone comprise entre 315 nm et 400 nm et au-delà, ou bien dans l'ensemble de ces deux zones. Ils sont le plus souvent formulés dans des compositions antisolaires qui se présentent sous forme d'émulsions huile-dans-eau ou eau-dans-huile Les filtres organiques, généralement lipophiles ou hydrophiles, sont présents sous forme dissoute, dans l'une ou l'autre de ces phases, en des quantités appropriées pour obtenir le facteur de protection solaire (FPS) recherché.

On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Outre leur pouvoir filtrant du rayonnement solaire, les composés photoprotecteurs doivent également présenter de bonnes propriétés cosmétiques, une bonne solubilité dans les solvants usuels et en particulier dans les corps gras tels que les huiles et les graisses, une bonne résistance à l'eau et à la transpiration (rémanence) ainsi qu'une photostabilité satisfaisante.

A cet égard, on connaît dans l'état de la technique notamment dans le brevet EP0716089 une famille de filtres UV-A particulièrement intéressante constituée par des dérivés silaniques ou siloxaniques d'acrylonitrile. Ces composés présentent de très bonnes propriétés filtrantes dans l'UV-A , une meilleure solubilité dans les solvants organiques usuels et notamment les corps gras tels que les huiles par rapport à leurs homologues non siliciés, ainsi que d'excellentes propriétés cosmétiques. Cependant la demanderesse a constaté que ces filtres UV-A présentaient une photostabilité insuffisante.

La demanderesse a découvert de manière surprenante et inattendue , une nouvelle famille de dérivés silaniques ou siloxaniques de phénylsulfone acrylonitrile présentant des propriétés filtrantes dans l'UV-A, une solubilité dans les solvants organiques usuels ainsi que des propriétés cosmétiques aussi performantes que leurs homologues dérivés siliciés d'acrylonitrile non phénylsufonés de l'art antérieur mais présentant aussi par rapport à ces composés une photostabilité substantiellement supérieure.

La présente invention a pour objet les dérivés ou mélanges de dérivés silaniques ou siloxaniques de phénylsulfone acrylonitrile de formule (1), (2) ou (3) définies ci-dessous.

La présente invention a également pour objet une composition cosmétique à usage topique, en particulier destinée à la photoprot ction de la peau et/ou des cheveux, contenant, dans un support cosmétiquement acceptable, au moins un composé ou un mélange de composés siloxaniques ou silaniques dérivés de phénysulfone acrylonitrile de formule (1), (2) ou (3) définies ci-dessous.

La présente invention a enfin pour objet une utilisation des composés de formule (1), (2) ou (3) dans une composition cosmétique pour la protection de la peau et/ou des cheveux contre le rayonnement solaire comme filtre UV-A.

Les dérivés siloxaniques ou silaniques de phenylsulfone acrylonitrile, sous forme isolée ou de mélange , correspondent à l'une des formules générales (1), (2) ou (3) suivantes :

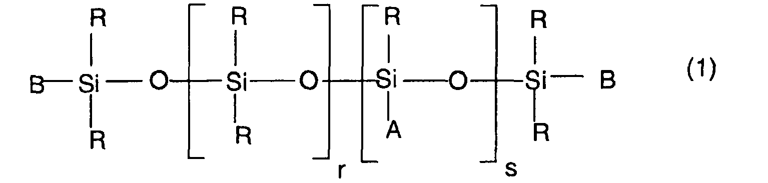

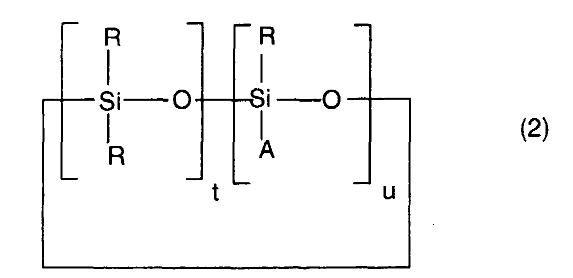

A-SiR'₁R'₂R'₃ (3)

dans lesquelles :
- A désigne un radical de formule (I) suivante : 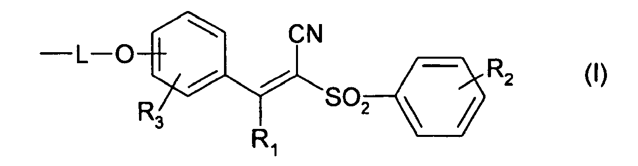
- R₁ désigne un atome d'hydrogène ; un radical alkyle en C₁-C₈ linéaire ou ramifié, saturé ou insaturé ; un radical phényle éventuellement substitué par un ou plusieurs radicaux alkyles ou alcoxy en C₁-C₈ linéaires ou ramifiés, saturés ou insaturés,
- R₂ désigne un atome d'hydrogène ; un radical alkyle en C₁-C₈, linéaire ou ramifié, saturé ou insaturé ; un radical alcoxy en C₁-C₈, linéaire ou ramifié, saturé ou insaturé ;
- R₃ désigne un radical alkyle en C₁-C₈ , linéaire ou ramifié, saturé ou insaturé ; un radical alcoxy en C₁-C₈ , linéaire ou ramifié , saturé ou insaturé ; H ;
- L est un radical divalent permettant l'accrochage du radical A sur la chaîne siliconée choisi parmi méthylène, éthylène ou un groupe répondant à l'une des formules (a), (a') ou (a") suivantes : 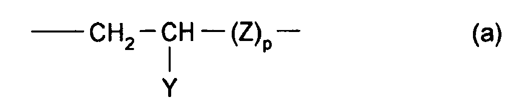 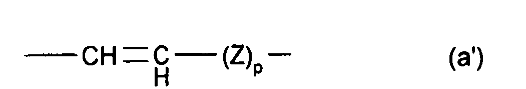 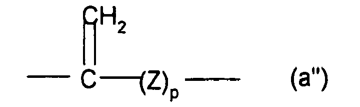
dans lesquelles :
- Z est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
- Y représente un atome d'hydrogène ; un radical hydroxyle ou un radical alkyle en C₁-C₈, linéaire ou ramifié, saturé ou insaturé,
- p est 0 ou 1,
- R, identiques ou différents désignent un groupe alkyle, linéaire ou ramifié, saturé ou insaturé en C₁-C₂₀, phényle, trifluoro-3,3,3 propyle ou un groupe triméthylsilyloxy ; au moins 80% en nombre des radicaux R étant méthyle,
- B, identiques ou différents, sont choisis parmi les radicaux R et le radical A,
- r est un nombre variant de 0 à 50 ,
- s est un nombre variant de 0 à 20 et si s est 0, au moins un des deux symboles B est A,
- u est un nombre variant 1 à 6 inclus,
- t est un nombre variant de 0 à 10 ;
- t+ u est supérieur ou égal à 3 .
- R'₁, R'₂, R'₃ , identiques ou différents, sont choisis parmi les radicaux alkyles en C₁-C₈, linéaires ou ramifiés, saturés ou insaturés ; le radical phényle ou le radical benzyle .

Bien que dans la formule (I) ci-dessus, seuls soient représentés les isomères dans lesquelles le substituant cyano est en position cis par rapport au substituant alcoxy-phényle, cette formule doit être comprise comme englobant également l'isomère trans correspondant.

Dans les formules (1) à (3) ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Le radical alkyle particulièrement préféré est le radical méthyle.

Dans les formules (1) à (3) ci-dessus, les radicaux alcoxy peuvent être linéaires ou ramifiés et choisis notamment au sein des radicaux méthoxy, éthoxy, n-propoxy, isopropoxy, n-butoxy et isobutoxy. Le radical alcoxy particulièrement préféré est le radical méthoxy.

Les diorganosiloxanes linéaires ou cycliques de formule (1) ou (2) rentrant dans le cadre de la présente invention, sont des oligomères ou polymères statistiques présentant de préférence au moins l'une, et encore plus préférentiellement l'ensemble, des caractéristiques suivantes :
- R est alkyle et encore plus préférentiellement méthyle,
- B est alkyle et encore plus préférentiellement méthyle (cas des composés linéaires de formule (1)),
- r varie de 0 à 10 inclusivement ; s varie de 0 à 6 inclusivement (cas des composés linéaires de formule (1)),
- t+u varie de 3 à 5 (cas des composés cycliques de formule (2)),
- R₁ désigne H,
- R₂ désigne H ,
- R₃ désigne H ,
- p vaut 1,
- Z désigne CH₂
- Y désigne H ou CH₃.

Parmi les diorganosiloxanes de formule (1) plus particulièrement préférés, on peut citer :
- le [4-[2-méthyl-3-[1,3,3,3-tetraméthyl-1-[(triméthylsilyl)oxy]-disiloxanyl]propyloxy]-phenyl]-2-benzènesulfonyl-acrylonitrile (composé A) de structure : 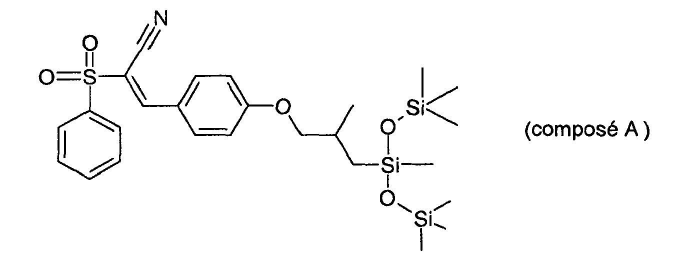
- un mélange de [4-[3-[1,3,3,3-tetraméthyl-1-[(triméthylsilyl)oxy]-disiloxanyl]-2-propènoxy]-phényl]-2-benzènesulfonyl-acrylonitrile (composé B) et de [4-[2-[1,3,3,3-tetraméthyl-1-[(triméthylsilyl)oxy]-disiloxanyl]-2-propènoxy]-phényl]-2-benzènesulfonyl-acrylonitrile (composé C) de structures suivantes (et plus particulièrement dont la rapport molaire composé B/composé C est 33/67) ; 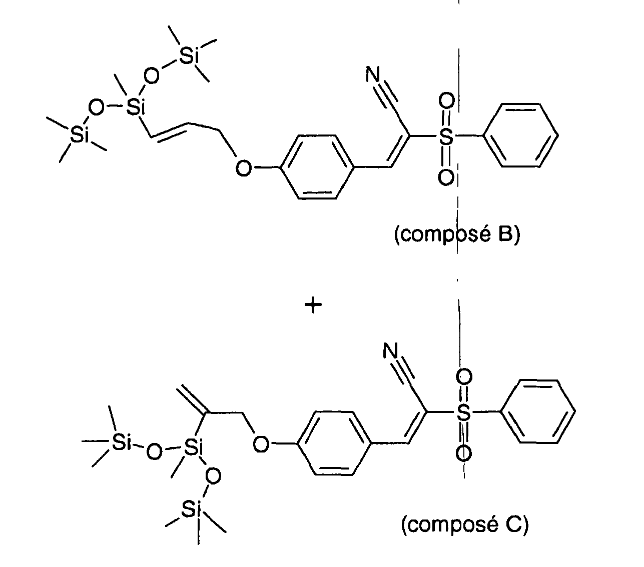
- un mélange de 3 α,ω-polydiméthylsiloxanes (Composés D, E, F) portant 2 groupements 4-(2-propènoxy-phényl)-2-benzènesulfonyl-acrylonitrile et/ou 4-(3-propènoxy-phényl)-2-benzènesulfonyl-acrylonitrile de structures suivantes :

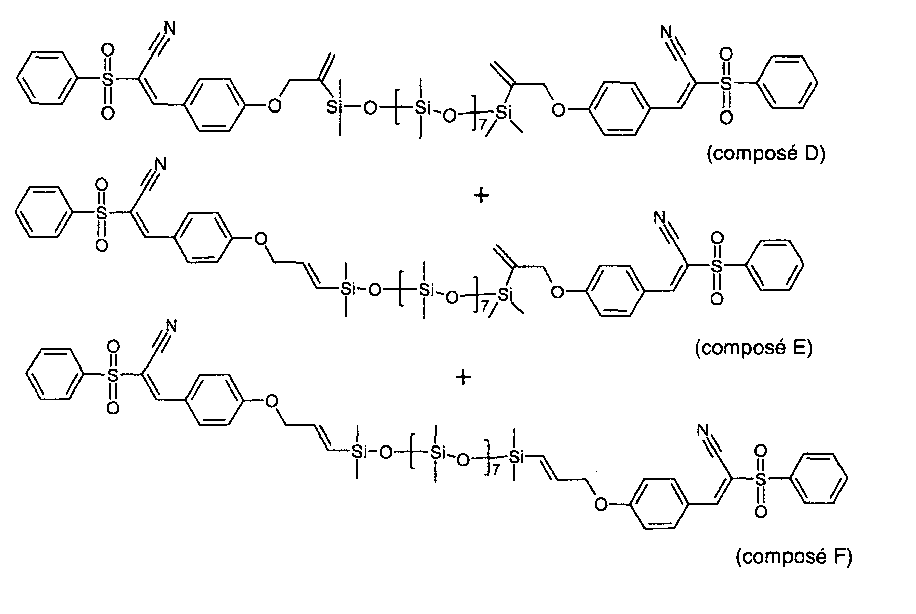

Les composés silaniques de formule (3) rentrant dans le cadre de la présente invention, présentent de préférence au moins l'une, et encore plus préférentiellement l'ensemble, des caractéristiques suivantes :
- R'₁ désigne méthyle
- R'₂ désigne méthyle
- R'₃ désigne méthyle
- R₁ désigne H
- R₂ désigne H
- R₃ désigne H
- p vaut 1,
- Z désigne CH₂
- Y désigne H ou CH₃.

Parmi les composés silaniques de formule (3) plus particulièrement préférés, on peut citer le 2-benzenesulfonyl-3-[4-(3-trimethylsilanyl-propoxy)-phenyl]-acrylonitrile de structure suivante :

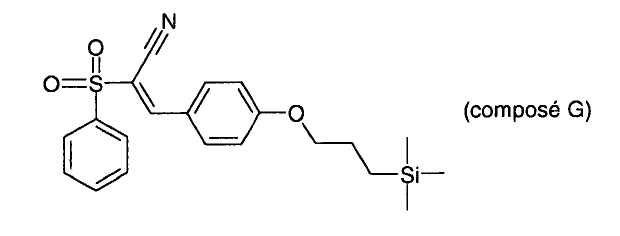

Pour préparer les dérivés de formule (1) ou (2), on peut procéder classiquement en mettant en oeuvre une réaction d'hydrosilylation à partir du dérivé siloxanique correspondant dans lequel, par exemple, tous les radicaux A sont des atomes d'hydrogène. Ce dérivé est dénommé par la suite dérivé à SiH.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3220972, US-A-3697473 et US-A-4340709.

Les dérivés à SiH correspondant respectivement aux composés de formules (1) et (2) peuvent être donc représentés par les formules 4) et (5) suivantes : 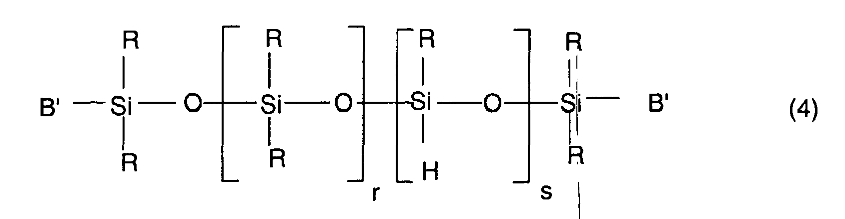 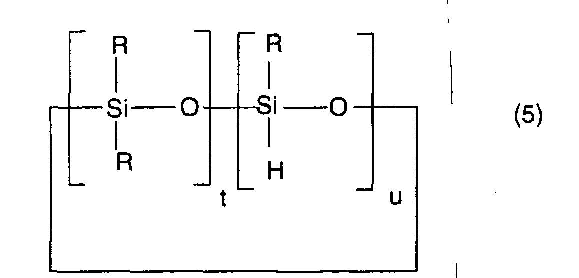 dans lesquelles :
- R, r, s, t et u ont la signification donnée ci-dessus pour les formules (1) et (2),
- B', identiques ou différents, sont choisis parmi les radicaux R et un atome d'hydrogène.

Afin de préparer les composés siloxaniques de l'invention de formules (1) ou (2) ci-dessus, on procède de la manière suivante : (schéma 1) : sur le dérivé à SiH de formule (4) ou (5) correspondant, on effectue une réaction d'hydrosilylation en présence d'une quantité catalytiquement efficace d'un catalyseur au platine sur un dérivé organique de phénylsulfone acrylonitrile de formule (I') suivante : 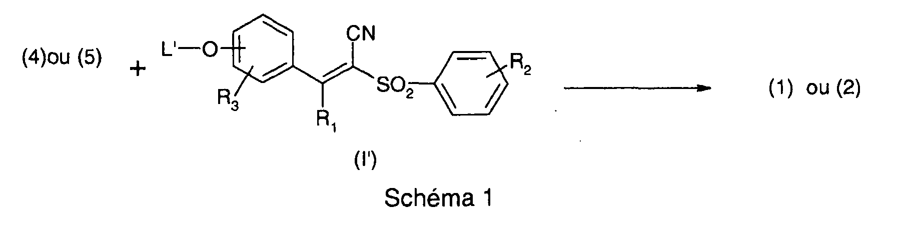 dans laquelle R₁, R₂ et R₃ ont la même signification qu'à la formule (I) ci-dessus et L' répond à l'une des deux formules (b) et (b') suivantes : 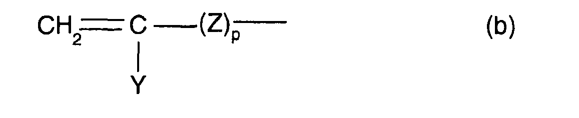 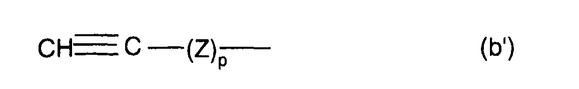 dans lesquelles Y, Z, p et q ont les mêmes significations qu'aux formules (a) et (a') ci-dessus.

La réaction d'hydrosilylation s'effectue donc selon l'une des deux réactions suivantes : 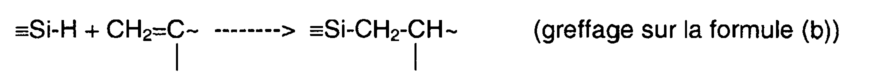 ou

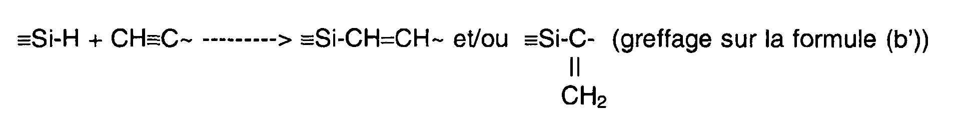

Les dérivés de formule (I') sont obtenus par condensation d'un halogénure d'alcène ou d'alcènyle sur un dérivé de formule (I"): 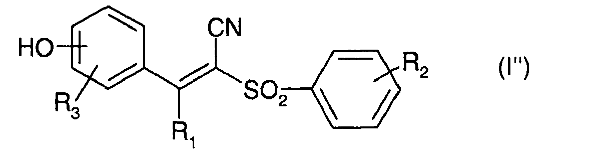 dans laquelle les radicaux R₁, R₂ et R₃ ont la même signification qu'aux formules (I) et (I') précédentes.

Les dérivés de formule (I") sont obtenus par condensation d'une hydroxy benzaldéhyde aromatique ou d'une hydroxy phénone avec un phénylsulfonyl acétonitrile dans le toluène en présence d'acétate de piperidinium comme catalyseur (condensation de Knoevenagel) selon le schéma suivant : 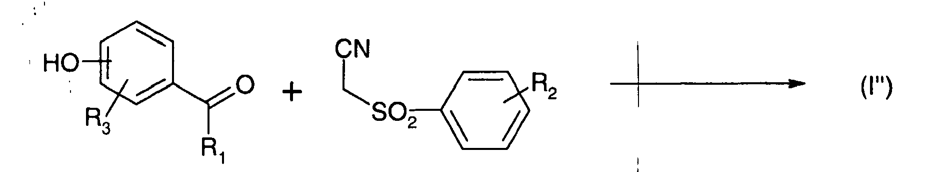 dans laquelle les radicaux R₁, R₂ et R₃ ont la même signification qu'aux formules (I) et (I').

Comme dérivés de benzaldéhyde aromatiques, on peut citer l'hydroxy-4 benzaldéhyde ou la vanilline qui sont des produits commerciaux. Comme dérivé de phénone, on peut citer l'hydroxy-4 acétophénone.

Comme dérivé phénylsulfonyl acétonitrile, on peut citer le benzènesulfonyl acétonitrile qui est un produit commercial.

Pour préparer les dérivés silaniques de formule (3), on peut utiliser un des deux schémas suivants:
(A) Voie où on fait réagir sur une hydroxy benzaldéhyde aromatique ou une hydroxy phénone un dérivé silanique halogéné en présence d'une base (réaction d'alkylation classique), la benzaldéhyde ou la phénone obtenue étant condensée avec un phénylsulfonyl acétonitrile dans le toluène en présence d'acétate de piperidinium comme catalyseur (condensation de Knoevenagel) selon le schéma 2 suivant : 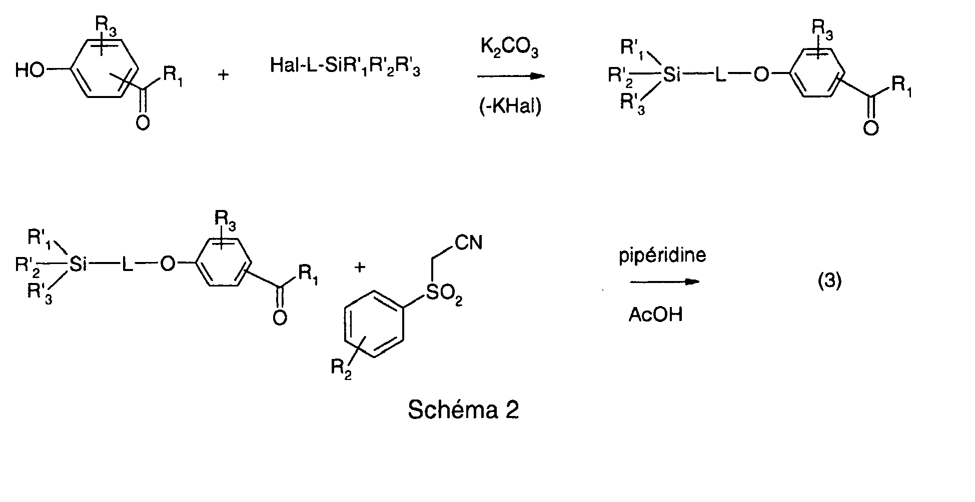 dans lequel L, R'₁ à R'₃ et R₁ à R₃ ont les indications indiquées ci-dessus pour les formules (3) et (I) définies ci-dessus et Hal représente un halogène et plus particulièrement le chlore.
(B) Voie qui consiste à partir du dérivé de formule (6) suivante dans laquelle R₁ à R₃ ont les mêmes significations qu'à la formule (I) ci-dessus et lui faire réagir, en présence d'une base (réaction d'alkylation classique), le dérivé de formule (7) dans laquelle L, R'₁ à R'₃ ont la même définition qu'à la formule (3) ci-dessus et Hal représente un halogène et plus particulièrement le chlore selon le schéma suivant :

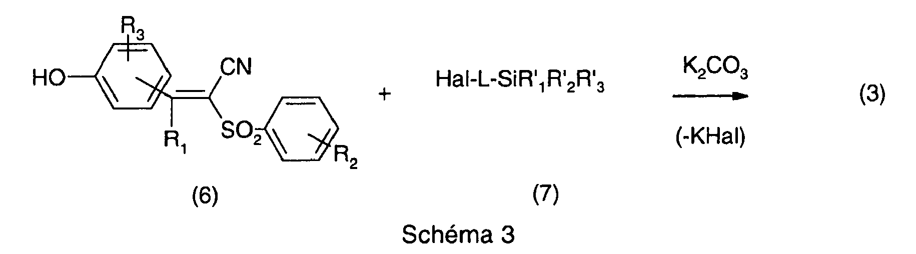

Comme dérivés d'halogénure silanique, on peut citer le chloropropyl triméthyl silane ou le chlorométhyl triméthyl silane vendus par la société WACKER.

Les composés utilisés dans les compositions selon l'invention présentent une bonne liposolubilité et peuvent ainsi être utilisés à des concentrations importantes dans les compositions cosmétiques, ce qui peut conférer aux compositions les contenant un indice de protection très élevé. Par ailleurs, cette bonne solubilité fait qu'ils se répartissent uniformément dans les supports cosmétiques classiques contenant au moins une phase grasse ou un solvant organique cosmétiquement acceptable et peuvent être ainsi appliqués sur la peau ou les cheveux pour former un film protecteur efficace.

Comme indiqué ci-dessus, les composés de formules (1), (2) ou (3) présentent un excellent pouvoir filtrant dans la gamme UV-A c'est-à-dire dans le domaine de longueurs d'ondes allant de 320 nm à 400 nm, présentent une photostabilité satisfaisante.

Le ou les composés de formule (1), (2) ou (3) sont de préférence présents dans les compositions cosmétiques à usage topique de la présente invention à raison de 0,1 % et 20 % en poids et en particulier à raison de 0,5 à 10 % en poids, par rapport au poids total de la composition cosmétique.

Les compositions cosmétiques, en particulier anti-solaires, de la présente invention peuvent contenir en outre un ou plusieurs filtres solaires organiques complémentaires actifs dans le domaine des UV-A et/ou UV-B.

Les filtres UV organiques complémentaires conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649; les dérivés de 4,4-diarylbutadiène tels que ceux décrits les demandes EP0967200, DE19746654, DE19755649, EP-A-1008586, EP1133980 et EP1133981 et leurs mélanges.

Comme exemples de filtres organiques complémentaires, on peut citer désignés ci-dessus sous leur nom INCI :

### Dérivés de l'acide para-aminobenzoique:

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone:

Benzophenone-1 vendu sous le nom commercial « UVINUL 400 » par BASF,
Benzophenone-2 vendu sous le nom commercial « UVINUL D50 » par BASF Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de la triazine :

Anisotriazine vendu sous le nom commercial «TINOSORB S » par CIBA GEIGY, Ethylhexyl triazone vendu notamment sous le nom commercial «UVINUL T150 » par BASF,
2,4,6-tris-(4'-amino benzalmalonate de diisobutyle)-s- triazine,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxanes à fonction benzalmalonate tels que le Polysilicone-15 vendu sous la dénomination commerciale « PARSOL SLX » par HOFFMANN LA ROCHE

### Dérivés de 4,4-diarylbutadiène :

- 1,1 -dicarboxy (2,2'-diméthyl-propyl)-4,4-diphénylbutadiène et leurs mélanges.

Les filtres UV organiques complémentaires plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
2,4,6-tris-(4'-amino benzalmalonate de diisobutyle)-s- riazine.
Methylene bis-Benzotriazolyl Tetramethylbutylphenol,
Drometrizole Trisiloxane,
Polysilicone 15,
1,1-dicarboxy (2,2'-diméthyl-propyl)-4,4-diphénylbutadiène
et leurs mélanges.

Les filtres UV complémentaires conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions cosmétiques selon l'invention peuvent contenir en outre un ou plusieurs pigments minéraux et en particulier des nanopigments d'oxydes métalliques, enrobés ou non, comme par exemple les nanopigments d'oxyde de titane sous forme amorphe ou cristallisée (rutile et/ou anatase), d'oxyde de fer, d'oxyde de zinc, d'oxyde de zirconium ou d'oxyde de cérium. Ces nanopigments ont une taille moyenne de particules comprise entre 5 nm et 100 nm, de préférence entre 10 nm et 50 nm et sont tous des agents photoprotecteurs UV connus.

Ces nanopigments peuvent être enrobés par des agents d'enrobage connus comme par exemple l'alumine et/ou le stéarate d'aluminium. De tels nanopigments enrobés ou non enrobés sont décrits par exemple dans les demandes de brevets EP-A-0 518 772 et EP-A-0 518 773.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les acides polyacryliques réticulés, les polymères d'acide acrylamidométhylpropane sulfonique (AMPS) comme le polyacrylamide/isoparaffine/laureth-7 (Sepigel 305), l'acide stéarique, les alcools gras, les gommes de xanthane, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets, mais nullement limitatifs, illustrant l'invention, vont maintenant être donnés.

### EXEMPLES DE PREPARATION

### EXEMPLE 1 :

### Synthèse du 2-Benzenesulfonyl-3-[4-(3-trimethylsilanyl-propoxy)-phenyl]-acrylonitrile (composé G) de formule (3) (avec R'₁ à R'₃ = CH₃, R₁ à R₃ = H, L est de formule (a) avec Y = H, Z = CH₂ et p = 1)

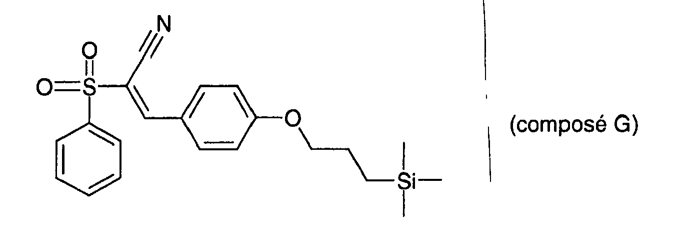

### a) Première étape : préparation du 4-(3-triméthylsilanyl-propyloxy)-benzaldéhyde :

Dans un mélange de 4-hydroxy benzaldéhyde (24,4 g, 0,2 mole) et de carbonate de potassium (30,4 g, 0,22 mole) dans 150 ml de DMF sec porté à 120°C sous azote, on ajoute goutte à goutte en 10 minutes du 3-chloropropyltriméthylsilane (33,14 g, 0,22 mole). On laisse 2 heures 30 minutes à 120-130°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide (0,2 mmHg), on obtient 40,5g (Rendement : 86 %) de 4-(3-triméthylsilanyl-propyloxy)-benzaldéhyde sous forme d'une huile incolore distillant à 110-114°C et utilisée telle quelle dans l'étape suivante.

### b) Deuxière étape : préparation du dérivé de l'exemple 1 :

Du phenylsulfonylacétonitrile (4g, 22.1 mmol) et le dérivé précédent (5,22g, 22.1 mmol) sont mis en solution dans 40 ml d'éthanol absolu et portés au reflux en présence d'une quantité catalytique de pipéridine (0,5 ml) pendant 48 heures.

Le milieu réactionnel est refroidi à température ambiante et le précipité formé est filtré et lavé par de l'éthanol froid. Après séchage au dessicateur, on obtient 8,82g (Rendement 82%) du dérivé de l'exemple 1 sous la forme d'un solide jaune pâle.

Point de fusion : 74°C

RMN ¹ H (DMSO-d6 , 400MHz, δ ppm) : 8.46 (s, 1 H) ; 8.04 (d, 2H) ; 8.01 (d, 2H) ; 7.81 (t, 1H) ; 7.73 (m, 2H) ; 7.15 (d, 2H) ; 4.06 (m, 2H) ; 1.73 (m, 2H) ; 0.58 (m, 2H) ; 0.01 (s, 9H)

RMN ¹³C (DMSO-d6 , 100MHz, δ ppm) : 165.5 ; 154.3 ; 139.9 ; 136.5 ; 135.6 ; 131.7; 129.5; 124.2; 117.3; 115.6; 110.7; 72.4; 24.8; 13.6; 0.

UV (EtOH), λₘₐₓ = 347 nm ; εₘₐₓ= 32 500 ; E1% = 813.

### EXEMPLE 2

### Synthèse du [4-[2-méthyl-3-[1,3,3,3-tetraméthyl-1-[(triméthylsilyl)oxy]-disiloxanyl]propyloxy]-phenyl]-2-benzènesulfonyl-acrylonitrile (composé A) de formule (1) avec R₁ à R₃ = H, s=1, r=0 et B=R=CH₃, L est de formule (a) avec Y = CH₃, Z = CH₂ et p = 1

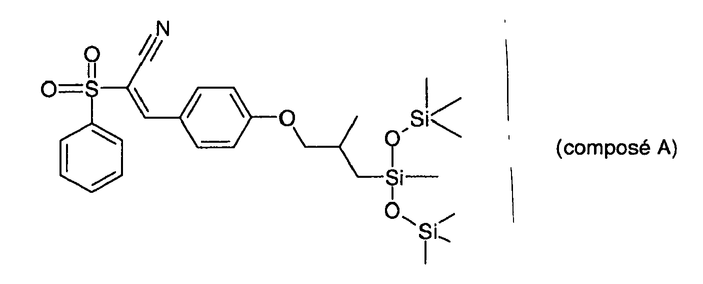

### a) Première étape : préparation du 2-benzènesulfonyl-3-[4-(2-méthyl-allyloxy)-phényl]-acrylonitrile :

Une solution de 4-(2-méthyl-allyloxy) benzaldéhyde (7,03 g, 40 mmole) et de phenylsulfonylacétonitrile (7,24g, 40 mmole) dans 70 ml d'éthanol absolu est portée au reflux en présence d'une quantité catalytique de pipéridine (0,5ml) pendant 48 heures. Le milieu réactionnel est refroidi à température ambiante et le précipité formé est filtré et lavé par de l'éthanol froid. Après séchage au dessicateur, on obtient 10,45g (Rendement 77%) de 2-benzènesulfonyl-3-[4-(2-méthyl-allyloxy)-phényl]-acrylonitrile sous la forme d'un solide jaune très pâle et utilisé tel quel dans l'étape suivante.

RMN ¹H (DMSO-d6 , 400MHz, δ ppm) : 8.44 (s, 1 H) ; 8.04 (d, 2H) ; 8.00 (d, 2H) ; 7.82 (m, 1H) ; 7.73 (m, 2H) ; 7.18 (d, 2H) ; 5.00 (d, 2H) ; 4.61 (s, 2H) ; 1.76 (s, 3H).

RMN ¹³C (DMSO-d6 , 100MHz, δ ppm) : 163.3 ; 152.6 ; 140.0 ; 138.2 ; 134.8 ; 133.7; 130.0; 127.8; 122.7; 115.8; 113.8; 112.8; 109.3; 71.3; 19.0.

UV (EtOH), λ ₘₐₓ = 345 nm ; ε ₘₐₓ = 25 000, E 1 % = 736.

### b) Deuxième étape : préparation du dérivé de l'exemple 2 :

A une solution du dérivé précédent (7,5 g, 22,1 mmol) et de catalyseur au platine (complexe à 3-3.5% poids de Pt dans du cyclovinylméthylsiloxane de Hüls Petrarch PC085, 50µl) dans 20 ml de toluène sec porté à 70°C sont ajoutés en 30 minutes de l'heptamethyltrisiloxane (6 g, 27 mmol). Le milieu réactionnel est maintenu 6 heures à cette température. Le mélange est ensuite concentré et l'huile obtenue est séparé par chromatographie sur colonne de gel de silice en éluant par le mélange heptane / acétate d'éthyle 4/1.

On obtient 4,8g (Rendement 55%) du dérivé de l'exemple 2 sous forme d'une huile jaune.

RMN ¹H (DMSO-d6 , 400MHz, δ ppm) : 8.43 (s, 1H) ; 8.01 (dd, 4H) ; 7.82 (m, 1H); 7.73 (m, 2H) ; 7.13 (d, 2H) ; 3.91-3.85 (m, 2H) ; 2.05 (m, 1H) ; 1.02 (d, 3H) ; 0.72 (dd, 1 H) ; 0.42 (m, 1 H) ; 0.07 (s, 18H) ; 0.04 (s, 3H).

RMN ¹³C (DMSO-d6 , 100MHz, δ ppm) : 162.1 ; 150.8 ; 136.4 ; 133 ; 132 ; 128.2 ; 126; 120.7; 113.7; 112; 107.3; 73 ; 26.5 ; 19.5; 17.4; 0; -1.0.

UV (EtOH), λₘₐₓ = 348 nm ; εₘₐₓ = 35 500, E 1 % = 632.

### EXEMPLE 3:

### Synthèse du mélange [4-[3-[1,3,3,3-tetraméthyl-1-[(triméthylsilyl)oxy]-disiloxanyl]-2-propènoxyl-phényl]-2-benzènesulfonyl-acrylonitrile (composé B) et [4-[2-[1,3,3,3-tetraméthyl-1-[(triméthylsilyl)oxy]-disiloxanyl]-2-propènoxyl-phényl]-2-benzènesulfonyl-acrylonitrile (composé C) de formule (1) avec R₁ à R₃ = H, s=1, r=0 et B=R=CH₃, L est de formule (a') avec Y = H, Z =CH₂ et p=1

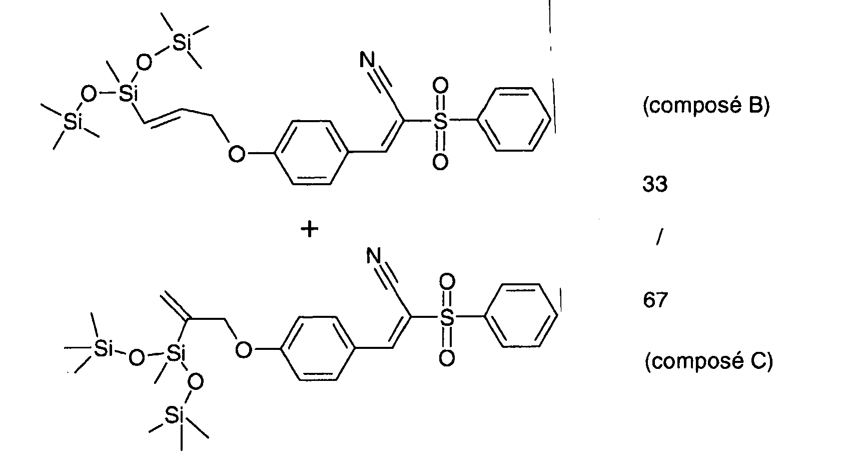

### a) Première étape : préparation du prop-2-ynyloxy-benzaldéhyde:

Une solution de 4-hydroxybenzaldéhyde (1 g, 8,2 mmol), de carbonate de potassium (2,27g, 16,4 mmol) et de bromure de propargyle (1,4 ml d'une solution à 80% dans le toluène) dans 50 ml d'acétone anhydre est chauffée au reflux pendant 5 heures. Le milieu réactionnel est refroidi à température ambiante, filtré et concentré. Le brut réactionnel obtenu est purifié par colonne de chromatographie sur gel de silice en éluant par le dichlorométhane. On obtient 1,31 g (Rendement quantitatif) de prop-2-ynyloxy-benzaldéhyde sous forme d'un solide blanc utilisé tel quel dans l'étape suivante.

RMN ¹H (DMSO-d6 , 400MHz, δ ppm) : 9.89 (s, 1H) ; 7.89 (d, 2H) ; 7.18 (d, 2H) ; 4.95 (s 2H) ; 3.65 (s, 1H).

### b) Deuxième étape : préparation du benzènesulfonyl-3-(4-prop-2-ynyloxy-phényl)-acrylonitrile :

Une solution du produit précédent (1,3 g, 8,12mmol) et de phénylsulfonylacétonitrile (1,47g, 8,1 mmol) dans 8 ml d'éthanol absolu est chauffée au reflux pendant 12 heures en présence d'une quantité catalytique de pipéridine (0,2 ml). Le milieu est ensuite refroidi à 0°C et filtré. Le solide obtenu est lavé par 2 ml d'éthanol absolu froid. On obtient 2,16 g (Rendement 82%) de benzènesulfonyl-3-(4-prop-2-ynyloxy-phényl)-acrylonitrile sous forme d'un solide blanc crème.

RMN ¹H (DMSO-d6 , 400MHz, δ ppm) : 8.47 (s, 1H) ; 8.08 (d, 2H) ; 8.06 (m, 2H) ; 7.83 (m, 1H) ; 7.76 (m, 2H) ; 7.20 (d, 2H) ; 4.95 (s 2H) ; 3.66 (s, 1H)

RMN ¹³C (DMSO-d6 , 100MHz, δ ppm) : 162.4 ; 153.0 ; 138.5 ; 135.2 ; 134.0 ; 130.5 ; 128.3 ; 123.7 ; 116.3 ; 114.1 ; 110.2 ; 79.4 ; 78.6 ; 56.4

### c) Troisième étape : préparation du produit de l'exemple 3 :

A une solution du produit précédent (1 g, 3,07 mmol) et de catalyseur au platine (complexe à 3-3.5% poids de Pt dans du cyclovinylméthylsiloxane de Hüls Petrarch PC085, 7µl) dans 10 ml de toluène sec à 70°C est ajouté en 5 minutes l'heptamethyltrisiloxane (1.02 ml, 3,7 mmol). Le milieu réactionnel est maintenu 19 heures à cette température. Le mélange est ensuite concentré et séparé par chromatographie sur colonne de gel de silice en éluant par le mélange heptane / acétate d'éthyle 4/1. On obtient 1,51g (Rendement 90%) du mélange des 2 dérivés de l'exemple 3 sous forme d'une huile jaune et dans le rapport 33 : 67 comme vu dans le spectre de RMN 1 H suivant :

RMN ¹H (DMSO-d6 , 400MHz, δ ppm) : 8.44 (s, 1H) ; 8.04 (m, 2H) ; 8.00 (d, 2H) ; 7.82 (t, 1H) ; 7.73 (t, 2H) ; 7.14 (t, 2H) ; 6.26 (dt, 0.33H, *Htrans) ;* 5.88 (m, 0.67H, *Hcis) ;* 5.85 (dt, 0.33H, *Htrans) ;* 5.62 (m, 0.67H, *Htrans) ;* 4.78 (dd, 0.66H, *CH*_{*2*} *trans) ;* 4.74 (s, 1.32H, *CH*_{*2*} *cis) ;* 0.15 (s, 2H) ; 0.07 (s, 14H) ; 0.04 (s, 6H)

RMN ¹³C (DMSO-d6 , 100MHz, δ ppm) : 163.3 ; 152.6 ; 144.7 ; 138.2 ; 134.8 ; 133.7; 130.05; 129.3; 127.8; 122.7; 115.8; 113.8; 109.3 ; 71.2 ; 1.7 ; 0.11.

UV (EtOH), λₘₐₓ: 345 nm ; ε ₘₐₓ = 33 000, E1% = 610.

### EXEMPLE 4 :

### Synthèse du mélange de trois composés de structure α,ω-polydiméthylsiloxane statistique (r=7) portant 2 groupements 4-(2-propènoxy-phényl)-2-benzènesulfonyl-acrylonitrile et/ou 4-(3-propènoxyphényl)-2-benzènesulfonyl-acrylonitrile de formule (1) avec R₁ à R₃ = H, s=0, r=7 et B=R=CH₃, L est de formule (a') avec Y = H, Z = CH₂ et p = 1

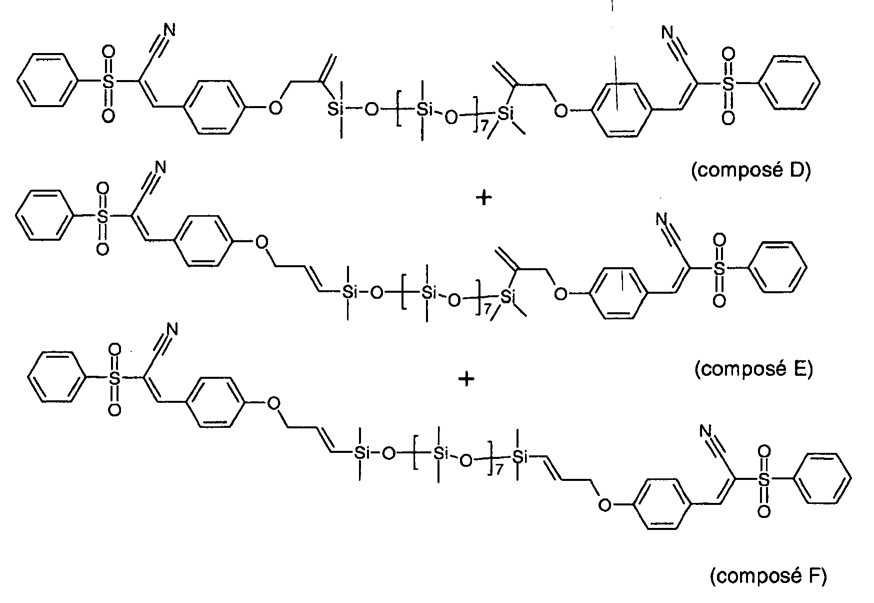

A une solution de benzènesulfonyl-3-(4-prop-2-ynyloxy-phényl)-acrylonitrile (1,15 g, 3,56 mmol) préparé à la deuxième étape de l'exemple 3 et de catalyseur au platine (complexe à 3-3.5% poids de Pt dans du cyclovinylméthylsiloxane de Hüls Petrarch PC085, 7µl) dans 10 ml de toluène sec à 70°C est ajouté en 7 minutes 1,51 ml d'α,ω-dihydro-polydiméthylsiloxane statistique (r=7) (2,1 méq en SiH). Le milieu réactionnel est maintenu 21 heures à cette température. Le mélange est ensuite concentré et séparé par chromatographie sur colonne de gel de silice en éluant par le mélange heptane / acétate d'éthyle 9/1. On obtient 1,21 g (Rendement 54%) du produit de l'exemple 4 sous forme d'une huile jaune :

RMN ¹³C (DMSO-d6 , 100MHz, δ ppm) : 163.3 ; 152.5 ; 146.0 ; 138.2 ; 134.8 ; 133.8; 130.0; 127.8; 126.8; 122.7; 115.7; 113.8; 109.3; 71.3; 1.03; 0.95; 0.4.
UV (EtOH), λₘₐₓ = 345 nm, E 1 % = 510.

### EXEMPLES DE FORMULATION

### EXEMPLE 1 :

- Mélange mono/distéarate de glycérol/stéarate de polyéthylèneglycol 100 OE (ARLACEL 165 FL-ICI) 1,0 g
- Alcool cétylique 0,5 g
- Acide stéarique d'huile de palme (STEARINE TP-STEARINERIE DUBOIS) 2.5 g
- Polydiméthylsiloxane (DOW CORNING 200 FLUID-DOW CORNING) 0.5 g
- Benzoate d'alcools en C₁₂/C₁₅ (WITCONOL TN- WITCO) 20 g
- Composé G 0.5 g
- Glycérine 5.0 g
- Phosphate d'alcool hexadécylique, sel de potassium (AMPHISOL K-HOFFMANN LA ROCHE) 1.0 g
- Acide polyacrylique (SYNTHALEN K- 3V) 0.3 g
- Hydroxypropylméthylcellusose (METHOCEL F4M- DOW CHEMICAL) 0.1 g
- Cyclopentadiméthylsiloxane (DC245-DOW CORNING) 2.0 g
- Triéthanolamine 0.8 g
- Conservateurs qs
- Eau déminéralisée qsp 100 g

### EXEMPLE 2 :

- Mélange d'alcool cétylstéarylique et d'alcool cétylstéarylique oxyéthyléné à 33 OE (80/20) (SINNOWAX AO-HENKEL) 7.0 g
- Mélange de mono- et de di-stéarate de glycérol (CERASYNT SD-V de ISP) 2.0 g
- Alcool cétylique 1.5 g
- Polydiméthylsiloxane (DOW CORNING 200 FLUID-DOW CORNING) 1.5 g
- Benzoate d'alcools en C₁₂/C₁₅ (WITCONOL TN- WITCO) 8.0 g
- Huile de vaseline 10.0 g
- Composé A 2.0 g
- Glycérine 10.0 g
- Conservateurs qs
- Eau déminéralisée qsp 100 g

## Revendications

1. Composé ou mélange de composés siloxaniques ou silaniques correspondant à l'une des formules générales (1), (2) ou (3) suivantes : 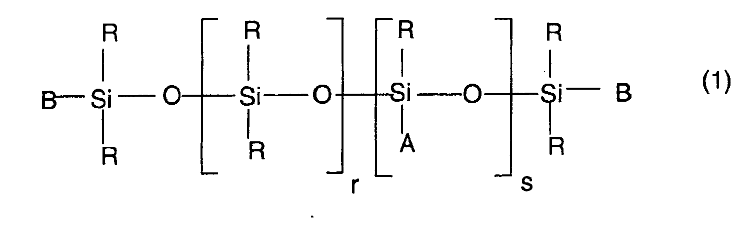 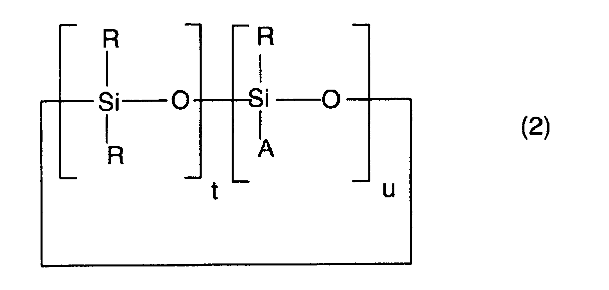
A-SiR'₁R'₂R'₃ (3)
dans lesquelles
- A désigne un radical de formule (I) suivante : 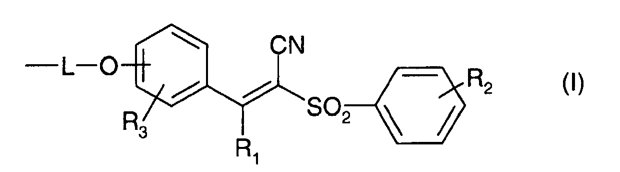 ainsi que sa forme isomère trans ;
- R₁ désigne un atome d'hydrogène ; un radical alkyle en C₁-C₈ linéaire ou ramifié, saturé ou insaturé ; un radical phényle éventuellement substitué par un ou plusieurs radicaux alkyles ou alcoxy en C₁-C₈ linéaires ou ramifiés, saturés ou insaturés,
- R₂ désigne un atome d'hydrogène ; un radical alkyle en C₁-C₈, linéaire ou ramifié, saturé ou insaturé ; un radical alcoxy en C₁-C₈, linéaire ou ramifié, saturé ou insaturé ,
- R₃ désigne un radical alkyle en C₁-C₈, linéaire ou ramifié, saturé ou insaturé ; un radical alcoxy en C₁-C₈, linéaire ou ramifié , saturé ou insaturé ; H ;
- L est un radical divalent permettant l'accrochage du radical A sur la chaîne sioxanique ou silanique choisi parmi méthylène, éthylène ou un groupe répondant à l'une des formules (a), (a') ou (a") suivantes : 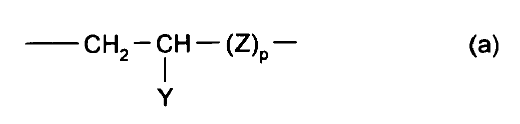 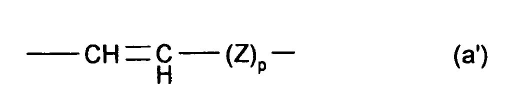 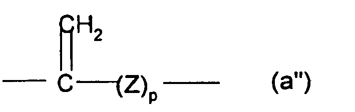
dans lesquelles :
- Z est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
- Y représente un atome d'hydrogène ; un radical hydroxyle ou un radical alkyle en C₁-C₈, linéaire ou ramifié, saturé ou insaturé,
- p est 0 ou 1,
- R, identiques ou différents désignent un groupe alkyle, linéaire ou ramifié, saturé ou insaturé en C₁-C₂₀, phényle, trifluoro-3,3,3 propyle ou un groupe triméthylsilyloxy ; au moins 80% en nombre des radicaux R étant méthyle,
- B, identiques ou différents, sont choisis parmi les radicaux R et le radical A,
- r est un nombre variant de 0 à 50 ,
- s est un nombre variant de 0 à 20 et si s est 0, au moins un des deux symboles B est A,
- u est un nombre variant 1 à 6 inclus,
- t est un nombre variant de 0 à 10 ;
- t+ u est supérieur ou égal à 3 .
- R'₁, R'₂, R'₃, identiques ou différents, sont choisis parmi les radicaux alkyles en C₁-C₈, linéaires ou ramifiés, saturés ou insaturés ; le radical phényle ou le radical benzyle.

2. Composé ou mélange de composés selon la revendication 1, correspondant à la formule (2) et répondant à au moins l'une et de préférence à l'ensemble des caractéristiques suivantes:
- R est alkyle et encore plus préférentiellement méthyle,
- t+u varie de 3 à 5,
- R₁ désigne H,
- R₂ désigne H,
- R₃ désigne H,
- p vaut 1,
- Z désigne CH₂
- Y désigne H ou CH₃.

3. Composé ou mélange de composés selon la revendication 1, correspondant à la formule (1) et répondant à au moins l'une et de préférence à l'ensemble des caractéristiques suivantes:
- R est alkyle et encore plus préférentiellement méthyle,
- B est alkyle et encore plus préférentiellement méthyle,
- r varie de 0 à 10 inclusivement
- s varie de 0 à 6 inclusivement,
- R₁ désigne H,
- R₂ désigne H,
- R₃ désigne H,
- p vaut 1,
- Z désigne CH₂
- Y désigne H ou CH₃.

4. Composé selon la revendication 3 , **caractérisé par le fait qu'**il s'agit du [4-[2-méthyl-3-[1,3,3,3-tetraméthyl-1-[(triméthylsilyl)oxy]-disiloxanyl]propyloxy]-phenyl]-2-benzènesulfonyl-acrylonitrile de structure :

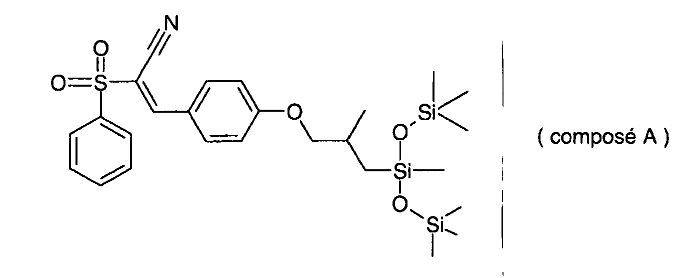

5. Mélange de composés selon la revendication 3 , **caractérisés par le fait qu'**il s'agit d'un mélange de [4-[3-[1,3,3,3-tetraméthyl-1-[(triméthylsilyl)oxy]-disiloxanyl]-2-propènoxy]-phényl]-2-benzènesulfonyl-acrylonitrile (composé B) et de [4-[2-[1,3,3,3-tetraméthyl-1-[(triméthylsilyl)oxy]-disiloxanyl]-2-propènoxy]-phényl]-2-benzènesulfonyl-acrylonitrile (composé C) de structures suivantes :

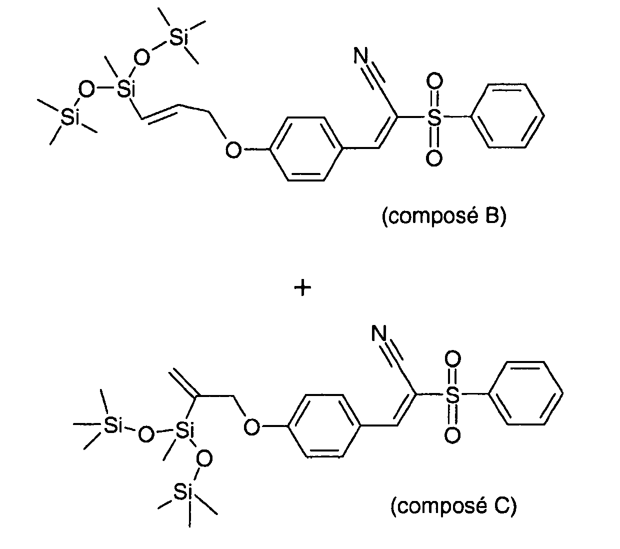

6. Mélange de composés selon la revendication 3 , **caractérisé par le fait qu'**il s'agit d'un mélange de 3 composés α,ω-polydiméthylsiloxanes portant 2 groupements 4-(2-propènoxy-phényl)-2-benzènesulfonyl-acrylonitrile et/ou 4-(3-propènoxy-phényl)-2-benzènesulfonyl-acrylonitrile de structures suivantes :

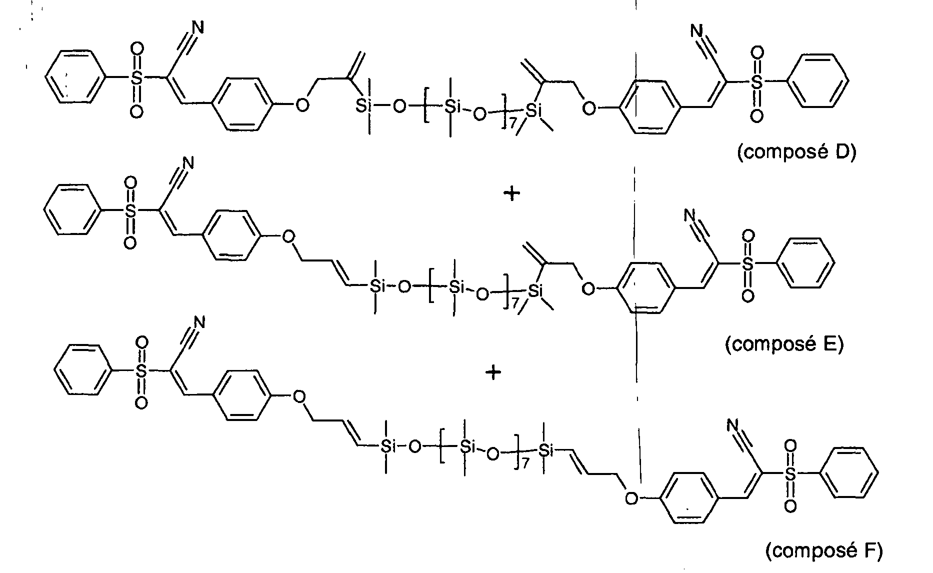

7. Composé ou mélange de composés selon la revendication 1, correspondant à la formule (3) et répondant à au moins l'une et de préférence à l'ensemble des caractéristiques suivantes:
- R'₁ désigne méthyle
- R'₂ désigne méthyle
- R'₃ désigne méthyle
- R₁ désigne H
- R₂ désigne H
- R₃ désigne H
- p vaut 1,
- Z désigne CH₂
- Y désigne H ou CH₃.

8. Composé selon la revendication 7, **caractérisé par le fait qu'**il s'agit du 2-benzenesulfonyl-3-[4-(3-trimethylsilanyl-propoxy)-phenyl]-acrylonitrile de structure suivante :

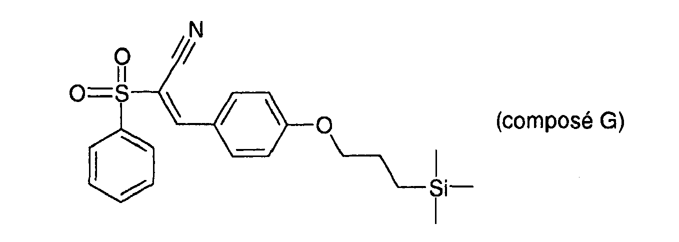

9. Composition cosmétique ou dermatologique à usage topique, en particulier pour la photoprotection de la peau et/ou des cheveux, **caractérisée par le fait qu'**elle comprend, dans un support cosmétiquement acceptable au moins un composé ou un mélange de composés de formule (1), (2) ou (3) tels que définis dans l'une quelconque des revendications précédentes

10. Composition selon la revendication 9, où le ou les composés de formule (1), (2) ou (3) sont présents à raison de 0,1 % et 20 % en poids et en particulier à raison de 0,5 à 10 % en poids, par rapport au poids total de la composition cosmétique.

11. Composition selon la revendication 9 ou 10, contenant en plus un ou plusieurs filtres solaires organiques complémentaires actifs dans le domaine des UV-A et/ou UV-B.

12. Composition selon la revendication 11 où les filtres UV organiques complémentaires sont choisis parmi les anthranilates ; les dérivés cinnamiques ; les dérivés de dibenzoylméthane ; les dérivés salicyliques ; les dérivés du camphre ; les dérivés de triazine ; les dérivés de la benzophénone ; les dérivés de β,β'-diphénylacrylate ; les dérivés de benzotriazole ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres autres que ceux de formule (1), (2) ou (3) ; les dimères dérivés d'α-alkylstyrène ; les dérivés de 4,4-diarylbutadiène.

13. Composition selon la revendication 12 où les filtres UV organiques complémentaires sont choisis parmi
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
2,4,6-tris-(4'-amino benzalmalonate de diisobutyle)-s- triazine,
Méthylène bis-Benzotriazolyl Tetramethylbutylphenol,
Drometrizole Trisiloxane,
Polysilicone 15,
1,1-dicarboxy (2,2'-diméthyl-propyl)-4,4-diphénylbutadiène,
et leurs mélanges.

14. Composition selon l'une quelconque des revendications 11 à 13, où le ou les filtres UV organiques complémentaires sont présents dans des proportions allant de 0,1 à 20% en poids par rapport au poids total de la composition, et de préférence allant de 0,2 à 15% en poids par rapport au poids total de la composition.

15. Composition selon l'une quelconque des revendications 9 4 à 14, contenant en plus un ou plusieurs pigments ou nanopigments d'oxydes métalliques, enrobés ou non,

16. Composition selon la revendication 15, où les oxydes métalliques sont choisis parmi les oxydes de titane sous forme amorphe, les oxydes de fer, les oxydes de zinc, les oxydes de zirconium, les d'oxydes de cérium et leurs mélanges.

17. Composition selon l'une quelconque des revendications **9** à 16, contenant en plus un ou plusieurs agents de bronzage et/ou de brunissage artificiel de la peau.

18. Composition selon l'une quelconque des revendications **9** à 17, contenant en plus un ou plusieurs adjuvants cosmétiques choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants.

19. Composition selon l'une quelconque des revendications 9 à 18, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

20. Composition selon l'une quelconque des revendications **9** à 18, **caractérisée par le fait qu'**il s'agit d'une composition de maquillage des ongles, des lèvres, des cils, des sourcils ou de la peau et qu'elle se présente sous forme solide ou pâteuse, anhydre ou aqueuse, d'une émulsion, d'une suspension ou d'une dispersion.

21. Composition selon l'une quelconque des revendications **9** à 18, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

22. Utilisation d'un composé ou d'un mélange de composés de formule (1), (2) où (3) tels que définis dans l'une quelconque des revendications 1 à 8 dans une composition cosmétique pour la protection de la peau et/ou des cheveux contre le rayonnement solaire comme agent filtrant les radiations UV de longueur d'onde allant de 320 à 400 nm.

## Claims

1. Siloxane or silane compound or mixture of compounds corresponding to one of the following general formulae (1), (2) or (3): 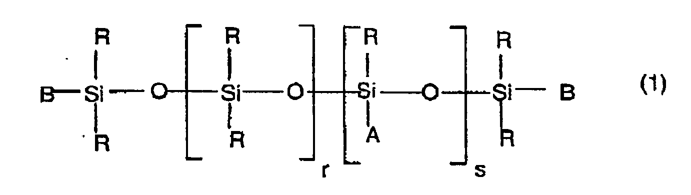 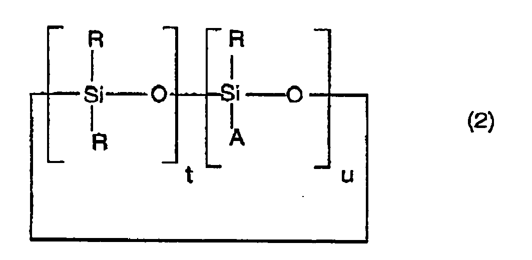
A-SiR'₁R'₂R'₃ (3)
in which:
- A denotes a radical of the following formula (I): 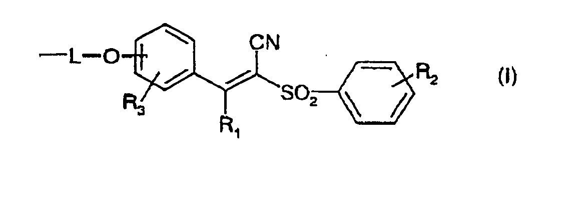 and its trans isomer form;
- R₁ denotes a hydrogen atom; a saturated or unsaturated, linear or branched C₁-C₈ alkyl radical; a phenyl radical which is optionally substituted with one or more saturated or unsaturated, linear or branched C₁-C₈ alkyl or alkoxy radicals,
- R₂ denotes a hydrogen atom; a saturated or unsaturated, linear or branched C₁-C₈ alkyl radical; a saturated or unsaturated, linear or branched C₁-C₈ alkoxy radical,
- R₃ denotes a saturated or unsaturated, linear or branched C₁-C₈ alkyl radical; a saturated or unsaturated, linear or branched C₁-C₈ alkoxy radical; H;
- L is a divalent radical allowing the attachment of the radical A onto the siloxane or silane chain chosen from methylene, ethylene or a group corresponding to one of the following formulae (a), (a') or (a"): 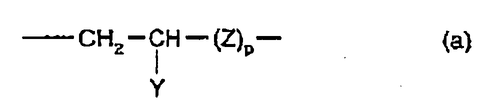 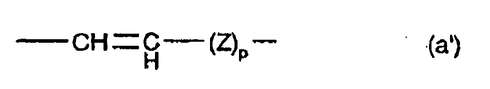 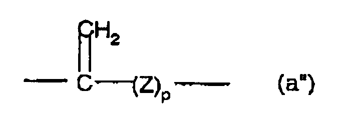
in which:
- Z is a saturated or unsaturated, linear or branched C₁-C₆ alkylene radical optionally substituted with a hydroxyl radical or a saturated or unsaturated, linear or branched C₁-C₈ alkyl radical,
- Y represents a hydrogen atom; a hydroxyl radical or a saturated or unsaturated, linear or branched C₁-C₈ alkyl radical,
- .p is 0 or 1,
- R, which are identical or different, denote a saturated or unsaturated, linear or branched C₁-C₂₀ alkyl group, a phenyl group, a 3,3,3-trifluoropropyl group or a trimethylsilyloxy group, at least 80% in numerical terms of the radicals R being methyl,
- B, which are identical or different, are chosen from the radicals R and the radical A,
- r is a number varying from 0 to 50,
- s is a number varying from 0 to 20 and if s is 0, at least one of the two symbols B is A,
- u is a number varying from 1 to 6 inclusive,
- t is a number varying from 0 to 10;
- t + u is greater than or equal to 3;
- R'₁, R'₂, R'₃, which are identical or different, are chosen from saturated or unsaturated, linear or branched C₁-C₈ alkyl radicals; the phenyl radical or the benzyl radical.

2. Compound or mixture of compounds according to Claim 1, corresponding to formula (2) and having at least one and preferably all of the following characteristics:
- R is alkyl and still more preferably methyl,
- t + u varies from 3 to 5,
- R₁ denotes H,
- R₂ denotes H,
- R₃ denotes H,
- p equals 1,
- Z denotes CH₂,
- Y denotes H or CH₃.

3. Compound or mixture of compounds according to Claim 1, corresponding to formula (1) and having at least one and preferably all of the following characteristics:
- R is alkyl and still more preferably methyl,
- B is alkyl and still more preferably methyl,
- r varies from 0 to 10 inclusive,
- s varies from 0 to 6 inclusive,
- R₁ denotes H,
- R₂ denotes H,
- R₃ denotes H,
- p equals 1,
- 2 denotes CH₂,
- Y denotes H or CH₃.

4. Compound according to Claim 3, **characterized in that** it is [4-[2-methyl-3-[1,3,3,3-tetramethyl-1-[(trimethylsilyl)oxy]disiloxanyl]-propyloxy]phenyl]-2-(benzenesulphonyl)acrylonitrile having the structure:

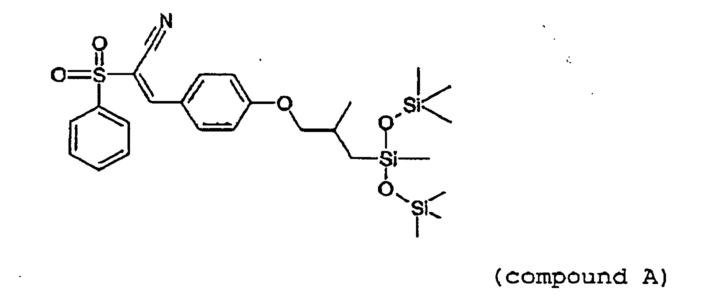

5. Mixture of compounds according to Claim 3, **characterized in that** it is a mixture of, [4-[3-[1,3,3,3-tetramethyl-1-[(trimethylsilyl)-oxy]disiloxanyl]-2-propenoxy]phenyl]-2-(benzenesulphonyl)acrylonitrile (compound B) and [4-[2-[1,3,3,3-tetramethyl-1-[(trimethylsilyl)oxy]-disiloxanyl]-2-propenoxy]phenyl]-2-(benzenesulphonyl)acrylonitrile (compound C) having the following structures:

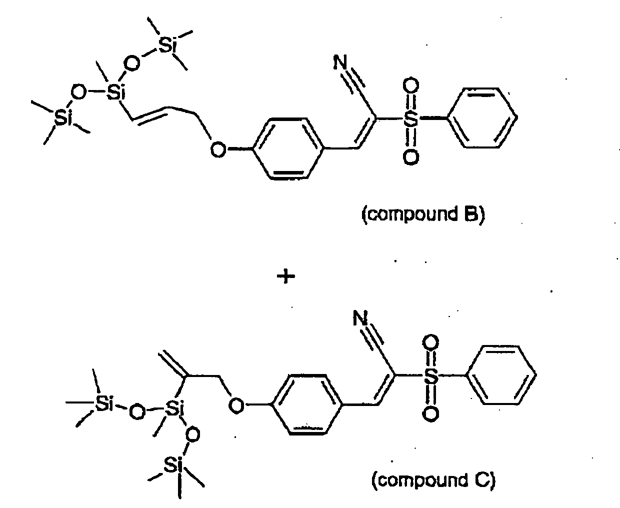

6. Mixture of compounds according to Claim 3, **characterized in that** it is a mixture of 3 α,ω-polydimethylsiloxane compounds carrying 2 groups 4-(2-propenoxyphenyl)-2-(benzenesulphonyl)acrylonitrile and/or 4-(3-propenoxyphenyl)-2-(benzenesulphonyl)-acrylonitrile having the following structures:

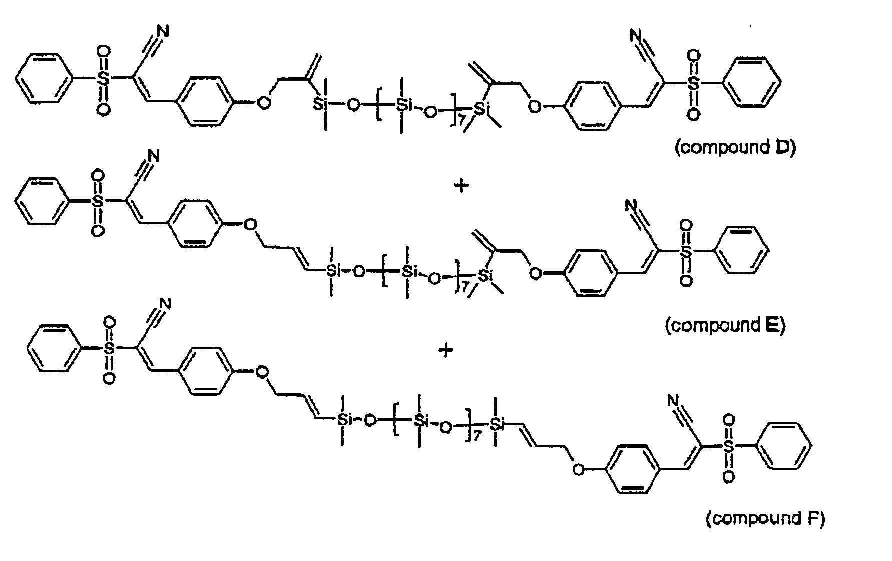

7. Compound or mixture of compounds according to Claim 1, corresponding to formula (3) and having at least one and preferably all of the following characteristics:
- R'₁ denotes methyl,
- R'₂ denotes methyl,
- R'₃ denotes methyl,
- R₁ denotes H,
- R₂ denotes H,
- R₃ denotes H,
- p equals 1,
- Z denotes CH₂,
- Y denotes H or CH₃.

8. Compound according to Claim 7, **characterized in that** it is 2-benzenesulphonyl-3-[4-(3-trimethylsilanylpropoxy)phenyl]acrylonitrile having the following structure:

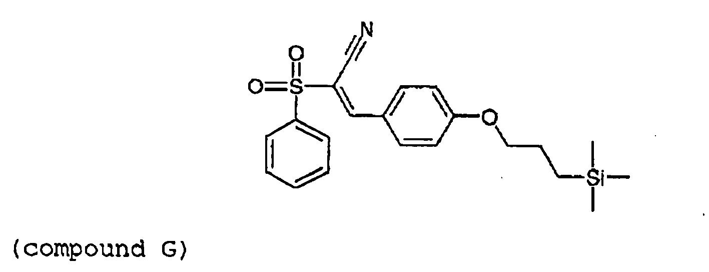

9. Cosmetic or dermatological composition for topical use, in particular for photoprotecting the skin and/or the hair, **characterized in that** it comprises, in a cosmetically acceptable carrier, at least one compound or a mixture of compounds of formula (1), (2) or (3) as defined in any one of the preceding claims.

10. Composition according to Claim 9, where the compound(s) of formula (1), (2) or (3) are present in an amount of 0.1% to 20% by weight, and in particular in an amount of 0.5 to 10% by weight, relative to the total weight of the cosmetic composition.

11. Composition according to Claim 9 or 10, containing in addition one or more additional organic sunscreens which are active in the UV-A and/or UV-B domain.

12. Composition according to Claim 11, where the additional organic UV-screening agents are chosen from anthranilates; cinnamic derivatives; dibenzoylmethane derivatives; salicylic derivatives; camphor derivatives; triazine derivatives; benzophenone derivatives; β,β'-diphenyl acrylate derivatives; benzotriazole derivatives; benzimidazole derivatives; imidazolines; bis-benzoazolyl derivatives; p-aminobenzoic acid (PABA) derivatives; inethylenebis(hydroxyphenylbenzotriazole) derivatives; screening polymers and screening silicones other than those of formula (1), (2) or (3); dimers derived from α-alkylstyrene; 4,4-diarylbutadiene derivatives.

13. Composition according to Claim 12, where the additional organic UV-screening agents are chosen from
Ethylhexyl Salicylate,
Butyl Methoxydibenzoylmethane,
Ethylhexyl Methoxycinnamate,
Octocrylene,
Phenylbenzimidazole Sulphonic Acid,
Terephthalylidene Dicamphor Sulphonic,
Benzophenone-3,
Benzophenone-4,
Benzophenone-5,
n-Hexyl 2-(4-diethylamino-2-hydroxybenzoyl)benzoate,
4-Methylbenzylidene camphor,
Disodium Phenyl Dibenzimidazole Tetra-sulphonate,
Anisotriazine,
Ethylhexyl triazone,
Diethylhexyl Butamido Triazone,
2,4,6-Tris(diisobutyl 4'-aminobenzalmalonate)-s-triazine,
Methylene bis-Benzotriazolyl Tetramethylbutylphenol,
Drometrizole Trisiloxane,
Polysilicone 15,
1,1-Dicarboxy(2,2'-dimethylpropyl)-4,4-diphenylbutadiene,
and mixtures thereof.

14. Composition according to any one of Claims 11 to 13, where the additional organic UV-screening agent(s) are present in proportions ranging from 0.1 to 20% by weight relative to the total weight of the composition, and preferably ranging from 0.2 to 15% by weight relative to the total weight of the composition.

15. Composition according to any one of Claims 9 to 14, containing in addition one or more pigments or nanopigments of metal oxides, coated or otherwise.

16. Composition according to Claim 15, where the metal oxides are chosen from titanium oxides in amorphous form, iron oxides, zinc oxides, zirconium oxides, cerium oxides and mixtures thereof.

17. Composition according to any one of Claims 9 to 16, containing in addition one or more agents for artificially bronzing and/or tanning the skin.

18. Composition according to any one of Claims 9 to 17, containing in addition one or more cosmetic adjuvants chosen from fatty substances, organic solvents, ionic or nonionic thickeners, demulcents, humectants, antioxidants, moisturizers, desquamating agents, anti-free-radical agents, antipollution agents, antibacterials, anti-inflammatory agents, depigmenting agents, propigmenting agents, opacifiers, stabilizers, emollients, silicones, antifoaming agents, insect repellents, perfumes, preservatives, anionic, cationic, nonionic, zwitterionic or amphoteric surfactants, substance P antagonists, substance CGRP antagonists, fillers, pigments, polymers, propellants, alkalinizing or acidifying agents.

19. Composition according to any one of Claims 9 to 18, **characterized in that** it is a protective composition for the human epidermis or an anti-sun composition and **in that** it is provided in the form of a nonionic vesicular dispersion, an emulsion, in particular an emulsion of the oil-in-water type, a cream, a milk, a gel, a gel cream, a suspension, a dispersion, a powder, a solid stick, a mousse or a spray.

20. Composition according to any one of claims 9 to 18, **characterized in that** it is a make-up composition for the nails, the lips, the eyelashes, the eyebrows or the skin and **in that** it is provided in an anhydrous or aqueous, solid or pasty form, or in the form of an emulsion, a suspension or a dispersion.

21. Composition according to any one of Claims 9 to 18, **characterized in that** it is a composition intended for protecting the hair against ultraviolet rays and **in that** it is provided in the form of a shaznpoo, a lotion, a gel, an emulsion or a nonionic vesicular dispersion.

22. Use of a compound or of a mixture of compounds of formula (1), (2) or (3) as defined in any one of Claims 1 to 8, in a cosmetic composition for protecting the skin and/or the hair against solar radiation as agent for screening UV radiation having a wavelength ranging from 320 to 400 nm.

## Patentansprüche

1. Siloxanverbindungen oder Silanverbindungen oder Gemische dieser Verbindungen, die einer der nachstehenden allgemeinen Formeln (1), (2) oder (3) entsprechen: 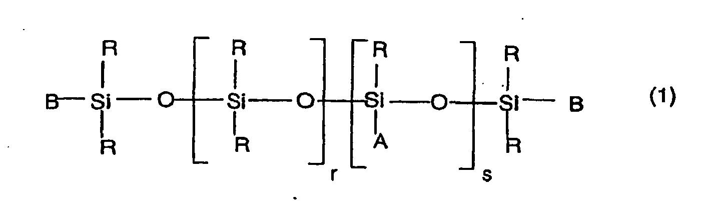 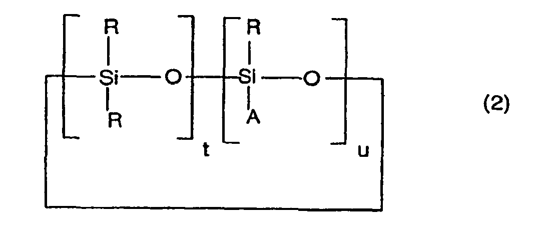
A-SiR'₁R'₂R'₃ (3)
worin bedeuten:
- eine Gruppe der folgenden Formel (I): 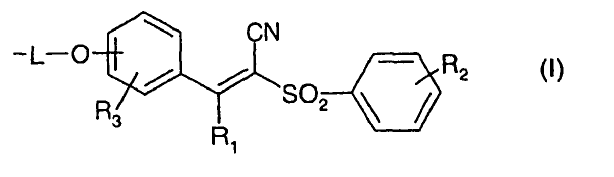 sowie ihr trans-Isomer;
- R₁ ein Wasserstoffatom; eine geradkettige oder verzweigte, gesättigte oder ungesättigte C₁₋₈-Alkylgruppe; eine gegebenenfalls mit einer oder mehreren geradkettigen oder verzweigten, gesättigten oder ungesättigten C₁₋₈-Alkylgruppen oder C₁₋₈-Alkoxygruppen substituierte Phenylgruppe,
- R₂ ein Wasserstoffatom; eine geradkettige oder verzweigte, gesättigte oder ungesättigte C₁₋₈-Alkylgruppe; eine geradkettige oder verzweigte, gesättigte oder ungesättigte C₁₋₈-Alkoxygruppe,
- R₃ eine geradkettige oder verzweigte, gesättigte oder ungesättigte c₁₋₈-Alkylgruppe; eine geradkettige oder verzweigte, gesättigte oder ungesättigte C₁₋₈-Alkoxygruppe; H
- L eine zweiwertige Gruppe, welche die Verknüpfung der Gruppe A mit der Siloxankette oder der Silankette erlaubt und ausgewählt ist unter Methylen, Ethylen oder einer Gruppe einer der nachstehenden Formeln (a), (a') oder (a"): 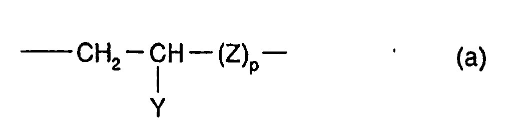 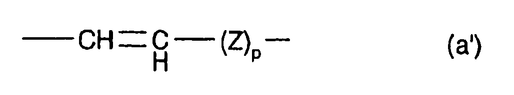 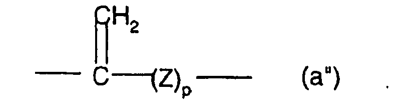 in denen darstellen:
- Z eine geradkettige oder verzweigte, gesättigte oder ungesättigte C₁₋₆-Alkylengruppe, die gegebenenfalls mit einer Hydroxylgruppe oder einer geradkettigen oder verzweigten, gesättigten oder ungesättigten C₁₋₈-Alkylgruppe substituiert ist,
- Y ein Wasserstoffatom; eine Hydroxylgruppe oder eine geradkettige oder verzweigte, gesättigte oder ungesättigte C₁₋₈-Alkylgruppe,
- p 0 oder 1,
- R₁, die gleich oder verschieden sind, eine geradkettige oder verzweigte, gesättigte oder ungesättigte C₁₋₂₀-Alkylgruppe, Phenyl, 3,3,3-Trifluorpropyl oder Trimethylsilyloxy, wobei mindestens 80 % der Gruppen R, bezogen auf die Anzahl der Gruppen R, Methyl sind,
- B, die gleich oder verschieden sind, Gruppen, die unter den Gruppen R und der Gruppe A ausgewählt sind,
- r eine Zahl von 0 bis 50,
- s eine Zahl von 0 bis 20, wobei, wenn s gleich 0 ist, mindestens eine der beiden Gruppen B gleich A ist,
- u eine Zahl von 1 bis einschließlich 6,
- t eine Zahl von 0 bis 10,
- wobei 1 + u gleich 3 oder größer als 3 ist,
- R'₁, R'₂, R'₃, die gleich oder verschieden sind, Gruppen, die ausgewählt sind unter geradkettigen oder verzweigten, gesättigten oder ungesättigten C₁₋₈-Alkylgruppen, der Phenylgruppe und der Benzylgruppe.

2. Verbindungen oder Gemische von Verbindungen nach Anspruch 1, die der Formel (2) entsprechen und mindestens eines der nachstehenden Merkmale und bevorzugt sämtliche nachstehenden Merkmale aufweisen:
- R ist eine Alkylgruppe und noch bevorzugter Methyl,
- t + u variiert von 3 bis 5,
- R₁ bezeichnet H,
- R₂ bezeichnet H,
- R₃ bezeichnet H,
- p hat den Wert 1,
- Z bezeichnet CH₂,
- Y bezeichnet H oder CH₃.

3. Verbindungen oder Gemische von Verbindungen nach Anspruch 1, die der Formel (1) entsprechen und mindestens eines der nachstehenden Merkmale und bevorzugt sämtliche nachstehenden Merkmale aufweisen:
- R ist eine Alkylgruppe und noch bevorzugter Methyl,
- B ist eine Alkylgruppe und noch bevorzugter Methyl,
- r varüert im Bereich von 0 bis einschließlich 10,
- s variiert im Bereich von 0 bis einschließlich 6,
- R₁ bezeichnet H,
- R₂ bezeichnet H,
- R₃ bezeichnet H,
- p hat den Wert 1,
- Z bezeichnet CH₂,
- Y bezeichnet H oder CH₃.

4. Verbindung nach Anspruch 3, **dadurch gekennzeichnet, dass** es sich um [4-[2-Methyl-3-[1,3,3,3-tetramethyl-1-[(trimethylsilyl)-oxy] -disiloxanyl] -propyloxy] -phenyl] -2 -benzolsulfonyl-acrylnitril der folgenden Struktur handelt:

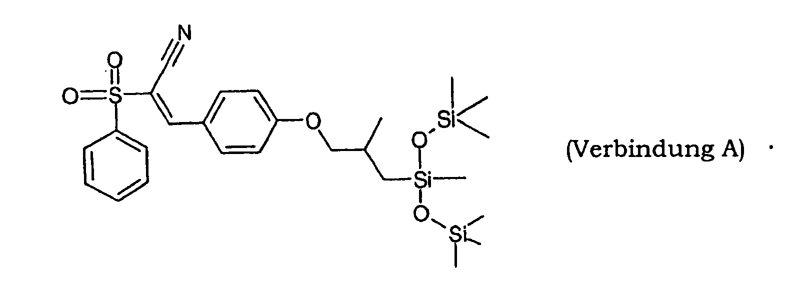

5. Gemisch von Verbindungen nach Anspruch 3, **dadurch gekennzeichnet, dass** es sich um ein Gemisch von [4-[3-[1,3,3,3-Tetramethyl-1-[(trimethylsilyl)oxyJ-disiloxanyl]-2-propenoxy]-phenyl]-2-benzolsulfonyl-acrylnitril (Verbindung B) und [4-[2-[1,3,3,3-Tetramethyl-1-[(trimethylsilyl)oxy]-disiloxanyl]-2-propenoxyJ-phenyl]-2-benzolsulfonyl-acrylnitril (Verbindung C) der folgenden Strukturen handelt:

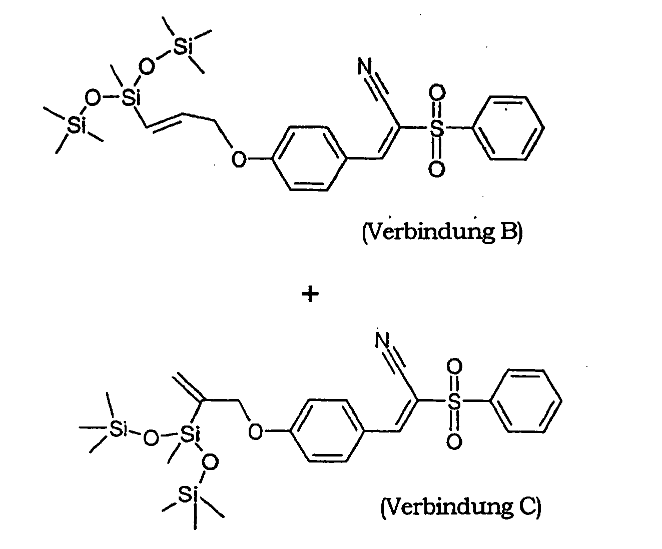

6. Gemisch von Verbindungen nach Anspruch 3, **dadurch gekennzeichnet, dass** es sich um ein Gemisch von 3 α,ω-Polydünethylsfloxaxl-Verbindungen handelt, die 2 4-(2-propenoxy-phenyl)-2-benzolsulfonyl-acrylnitril- und/oder 4-(3-propenoxy-phenyl)-2-benzolsulfonyl-acrylnitril-Gruppen aufweisen und die nachstehenden Strukturen besitzen:

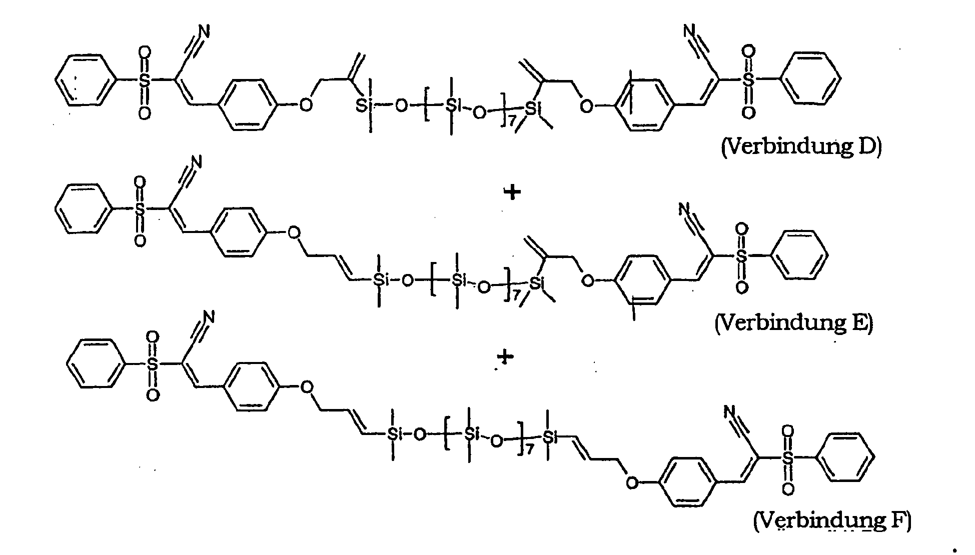

7. Verbindungen oder Gemische von Verbindungen nach Anspruch 1, die der Formel (3) entsprechen und mindestens eines der nachstehenden Merkmale und bevorzugt sämtliche nachstehenden Merkmale aufweisen:
- R'₁ bezeichnet Methyl,
- R'₂ bezeichnet Methyl,
- R'₃ bezeichnet Methyl,
- R₁ bezeichnet H,
- R₂ bezeichnet H,
- R₃ bezeichnet H,
- p hat den Wert 1,
- Z bezeichnet CH₂,
- Y bezeichnet H oder CH₃.

8. Verbindung nach Anspruch 7, **dadurch gekennzeichnet, dass** es sich um 2-Benzolsulfonyl-3-[4-(3-trimethylsilanyl-propoxy)-phenyl]-acrylnitril der nachstehenden Struktur handelt:

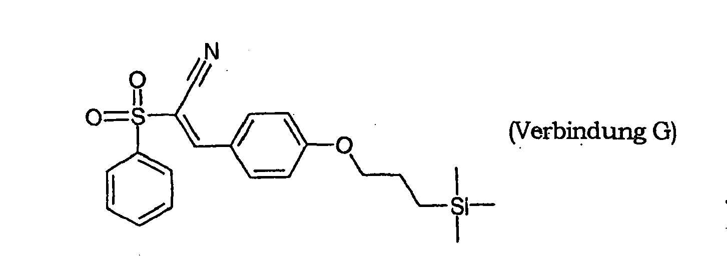

9. Kosmetische oder dermatologische Zusammensetzung zur topischen Anwendung, insbesondere zum Lichtschutz der Haut und/oder der Haare, **dadurch gekennzeichnet, dass** sie in einem kosmetisch akzeptablen Träger mindestens eine Verbindung oder ein Gemisch von Verbindungen der Formel (1), (2) oder (3), wie sie in einem der vorhergehenden Ansprüche definiert wurden, enthält.

10. Zusammensetzung nach Anspruch 9, bei der die Verbindung(en) der Formel (1), (2) oder (3) in einem Mengenanteil von 0,1 bis 20 Gew.-% und insbesondere in einem Mengenanteil von 0,5 bis 10 Gew.-%, bezogen auf das Gesamtgewicht der kosmetischen Zusammensetzung, vorliegen.

11. Zusammensetzung nach Anspruch 9 oder 10, die ein oder mehrere zusätzliche organische Sonnenfilter enthält, die im UV-A-Bereich und/oder im UV-B-Bereich wirksam sind.

12. Zusammensetzung nach Anspruch 11, bei der zusätzliche organische UV-Filter ausgewählt sind unter Anthranilaten; Zimtsäurederivaten; Derivaten von Dibenzoylmethan; Salicylsäurederivaten; Campherderivaten; Triazinderivaten; Benzophenonderivaten; Derivaten von β, β'-Diphenylacrylat; Benzotriazolderivaten; Benzimidazolderivaten; Imidazolinen; Bis-benzoazolyl-Derivaten; Derivaten von p-Aminobenzoesäure (PABA); Derivaten von Methylen-bis (hydroxyphenylbenzotriazol); polymeren Filtern und Siliconfiltern, die von denen der Formel (1), (2) oder (3) verschieden sind; von α-Alkylstyrolen abgeleiteten Dimeren; Derivaten von 4,4-Diarylbutadienen.

13. Zusammensetzung nach Anspruch 12, bei der die zusätzlichen organischen UV-Filter ausgewählt sind unter
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,
Benzophenone-3,
Benzophenone-4,
Benzophenone-5,
2-(4-Diethylamino-2-hydroxybenzoyl)-benzosäure-n-hexylester,
4-Methylbenzylidene Camphor,
Disodium Phenyl Dibenzimidazole Tetra-sulfonate,
Anisotriazine,
Ethylhexyl Triazone,
Diethylhexyl Butamido Triazone,
2,4,6-Tris diisobutyl-(4'-aminobenzalmalonat)-s-triazin,
Methylene bis-Benzotriazolyl Tetramethylbutylphenol,
Drometrizole Trisiloxane,
Polysilicone 15,
1,1-Dicarboxy-(2,2'-dimethyl-propyl)-4,4-Diphenylbutadien
sowie Gemischen dieser Verbindungen.

14. Zusammensetzung nach einem der Ansprüche 11 bis 13, bei der das oder die zusätzlichen organischen UV-Filter in Mengenanteilen von 0,1 bis 20 Gew.-%, bezogen auf das Gesamtgewicht der Zusammensetzung, und bevorzugt in Mengenanteilen von 0,2 bis 15 Gew.-%, bezogen auf das Gesamtgewicht der Zusammensetzung, vorliegen.

15. Zusammensetzung nach einem der Ansprüche 9 bis 14, die ferner ein oder mehrere Pigmente oder Nanopigmente von Metalloxiden enthält, die gegebenenfalls ummantelt sind.

16. Zusammensetzung nach Anspruch 15, bei der die Metalloxide ausgewählt sind unter Titanoxiden in amorpher Form, Eisenoxiden, Zinkoxiden, Zirconiumoxiden, Ceroxiden und Gemischen dieser Verbindungen.

17. Zusammensetzung nach einem der Ansprüche 9 bis 16, die ferner ein oder mehrere Mittel zur künstlichen Bräunung und/oder Braunfärbung der Haut enthält.

18. Zusammensetzung nach einem der Ansprüche 9 bis 17, die ferner ein oder mehrere kosmetische Hilfsstoffe enthält, die ausgewählt sind unter Fettstoffen, organischen Lösungsmitteln, ionischen oder nichtionischen Verdickungsmitteln, reizlindernden Mitteln, Befeuchtungsmitteln, Antioxidantien, hydratisierenden Mitteln, Desquamationsmitteln, Mitteln gegen freie Radikale, Mitteln gegen umweltbedingte Verschmutzung, antibakteriellen Mitteln, entzündungshemmenden Mitteln, Depigmentierungsmitteln, die Pigmentierung fördernden Mitteln, Trübungsmitteln, Stabilisatoren, Emollientien, Siliconen, Antischaummitteln, Insektenrepellents, Parfums, Konservierungsmitteln, anionischen, kationischen, nichtionischen, zwitterionischen oder amphoteren grenzflächenaktiven Mitteln, Antagonisten der Substanz P, Antagonisten der Substanz CGRP, Füllstoffen, Pigmenten, Polymeren, Treibmitteln, Mitteln zum Alkalischmachen oder Mitteln zum Ansäuern.

19. Zusammensetzung nach einem der Ansprüche 9 bis 18, **dadurch gekennzeichnet, dass** es sich um eine Zusammensetzung zum Schutz der menschlichen Epidermis oder um eine Sonnenschutzzusammensetzung handelt und die Zusammensetzung in Form einer nichtionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stiftes, eines Schaums oder eines Sprays vorliegt.

20. Zusammensetzung nach einem der Ansprüche 9 bis 18, **dadurch gekennzeichnet, dass** es sich um eine Zusammensetzung zum Schminken der Nägel, der Lippen, der Augenbrauen, der Wimpern oder der Haut handelt und die Zusammensetzung in fester oder pastoser Form, wasserfrei oder wasserhaltig, in Form einer Emulsion, einer Suspension oder einer Dispersion vorliegt.

21. Zusammensetzung nach einem der Ansprüche 9 bis 18, **dadurch gekennzeichnet, dass** es sich um eine Zusammensetzung zum Schutz der Haare gegen ultraviolette Strahlung handelt und die Zusammensetzung in Form eines Shampoos, einer Lotion, eines Gels, einer Emulsion oder einer nichtionischen Visikeldispersion vorliegt.

22. Verwendung einer Verbindung oder eines Gemisches von Verbindungen der Formel (1), (2) oder (3) wie in einem der Ansprüche 1 bis 8 definiert in einer kosmetischen Zusammensetzung zum Schutz der Haut und/oder der Haare gegen Sonnenstrahlung als Wirkstoff, der W-Strahlung einer Wellenlänge von 320 bis 400 nm ausfiltert.
